# FASCICULE DE BREVET EUROPEEN

(11) **EP 0 375 005 B1**
(45) Date de publication et mention de la délivrance du brevet: **21.09.1994**
(21) Numéro de dépôt: 89203080.0
(22) Date de dépôt: 05.12.1989
(51) Int. Cl.: C07D 333/16, C08G 61/12, H01B 1/12, H01M 4/62

(54) **Thiophènes substitués, polymères conducteurs dérivés de ces thiophènes, procédé pour leur obtention et dispositifs contenant ces polymères**
Substituierte Thiophene, leitfähige Polymere von diesen Thiophenen, Verfahren zu deren Herstellung und Vorrichtungen, enthaltend diese Polymere
Substituted thiophenes, conductive polymers derived from these thiophenes, process for their preparation and devices containing these polymers

(30) Priorité: 16.12.1988 FR 8816784
(43) Date de publication de la demande: 27.06.1990
(73) Titulaire: SOLVAY (Société Anonyme), B-1050 Bruxelles (BE)
(72) Inventeur: Garreau, Robert, F-95200 Sarcelles (FR); Roncali, Jean, F-93260 Les Lilas (FR); Lemaire, Marc, F-92000 Nanterre (FR); Korri, Hafsa, Paris 14ème (FR); Garnier, Francis, F-94500 Champigny (FR); Hannecart, Etienne, B-1980 Tervuren (BE)
(74) Mandataire: Nichels, William

(56) Documents cités:
- EP-A- 0 203 438
- EP-A- 0 240 063
- EP-A- 0 253 594
- EP-A- 0 323 656
- New J. Chem., 1989, vol. 13, n 12-1989, p. 863-866

## Description

La présente invention concerne des thiophènes substitués par un groupement contenant une fonction éther et un radical aliphatique ou aromatique au moins partiellement fluoré. L'invention concerne également les polymères conducteurs d'électricité contenant des unités récurrentes dérivées de ces thiophènes substitués, ainsi qu'un procédé pour la fabrication des thiophènes substitués et un procédé pour la fabrication des polymères et les dispositifs électroconducteurs contenant ces polymères.

Des polymères conducteurs d'électricité dérivés de monomères de formule générale : 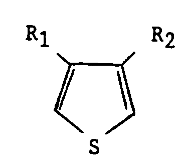
sont connus et ont notamment été décrits dans la demande EP-A-0253594 (Cookson Group); dans cette formule générale, R₁ peut représenter entre autres un groupement aryloxyalkyl ou un groupement de formule -(CH₂)ₙ-O-(CH₂CH₂)ₚ-O-R₄ dans laquelle R₄ représente un groupement alkyle contenant de 1 à 6 atomes de carbone et R₂ représente un atome d'hydrogène, un atome d'halogène ou un groupement aminé.

De même, dans la demande EP-A-203438 (Allied Corporation), on a décrit des polymères conducteurs d'électricité dérivés de monomères de formule générale : 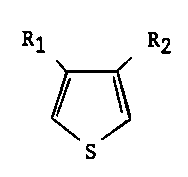
dans laquelle R₁ peut représenter entre autres un groupement alkoxy, aryloxy, alkoxyalkyle ou alkyle substitué par un groupement époxy, halo, acide carboxylique et R₂ représente un atome d'hydrogène ou un radical méthyle.

Toutefois, certaines applications électriques, telles que la réalisation de dispositifs (écrans d'affichage, commutateurs, éléments de mémoire...) basés sur l'électrochromisme (impliquant une modification des propriétés d'absorption ou de transmission de la lumière du matériau mis en oeuvre, induite par une variation de la tension externe appliquée), d'électrodes de batteries rechargeables, de cellules photovoltaïques, de cellules électrochimiques, des dispositifs d'absorption des ondes électromagnétiques, etc. exigent des polymères conducteurs aux propriétés particulières.

Ces propriétés particulières sont notamment la réversibilité électrochimique la plus complète et la stabilité la plus élevée possibles du cycle d'oxydo-réduction entre les formes oxydée et réduite du système polymère-agent dopant, ainsi qu'une variation importante des caractéristiques spectrales obtenue avec une variation de potentiel très faible, une bonne conductivité électrique et des absorptions importantes dans le domaine des rayonnements infrarouges proches et à haute fréquence.

La présente invention vise à fournir une nouvelle famille de thiophènes substitués permettant notamment d'obtenir des polymères conducteurs d'électricité qui présentent les propriétés particulières susmentionnées à un degré élevé.

L'invention concerne à cet effet des monomères dérivés de thiophènes substitués de formule générale : 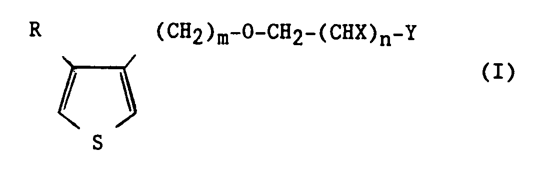
dans laquelle :
- R représente un atome d'hydrogène, un atome d'halogène ou un groupement aliphatique contenant de 1 à 4 atomes de carbone,
- X représente un atome d'hydrogène ou un atome de fluor,
- Y représente un groupement aliphatique ou aromatique au moins partiellement fluoré,
- m représente un nombre entier égal ou supérieur à 2,
- n représente un nombre entier tel que 0 ≦ n ≦ 7.

### Habituellement :

- R représente un atome d'hydrogène,
- X représente un atome d'hydrogène ou un atome de fluor,
- Y représente :
   . un radical phényle substitué au moins par un atome de fluor, par un groupement -CF₃, un groupement -CH₂F ou un groupement CHF₂, ou
   . un groupement aliphatique de formule générale -(CF₂)ₚ-CF₃ dans laquelle p représente un nombre entier tel que 0 ≦ p ≦ 16,
- m représente un nombre entier égal ou supérieur à 2,
- n représente un nombre entier tel que 0 ≦ n ≦ 7.

### Généralement :

- R représente un atome d'hydrogène,
- X représente un atome d'hydrogène,
- Y représente :
   . un radical phényle substitué par un atome de fluor, par un groupement -CF₃ ou entièrement substitué par des atomes de fluor, ou
   . un groupement aliphatique de formule générale -(CF₂)ₚ-CF₃ dans laquelle p représente un nombre entier tel que 0 ≦ p ≦ 12,
- m représente un nombre entier égal à 2 ou 3,
- n représente un nombre entier tel que 0 ≦ n ≦ 2.

### De manière préférée :

- R représente un atome d'hydrogène,
- X représente un atome d'hydrogène,
- Y représente :
   . un radical phényle substitué par un atome de fluor en para, par un groupement -CF₃ en para ou entièrement substitué par des atomes de fluor, ou
   . un groupement aliphatique de formule générale -(CF₂)ₚ-CF₃ dans laquelle p représente un nombre entier tel que 0 ≦ p ≦ 8,
- m est égal à 2,
- n représente un nombre entier égal à 0 ou 1.

### De manière particulièrement préférée :

- R représente un atome d'hydrogène,
- X représente un atome d'hydrogène,
- Y représente un radical phényle substitué par un atome de fluor en para,
- m est égal à 2,
- n est égal à 0.

Ce produit correspond au 4-fluorobenzyl-2-(3-thiényl)éthyléther.

Les thiophènes substitués selon l'invention peuvent être synthétisés selon des méthodes connues, par exemple par réaction des composés de formules générales : 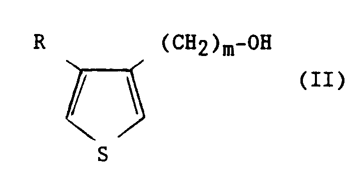
avec

Q-CH₂-(CHX)ₙ-Y (III)

dans lesquelles R, X, Y, m, n sont tels que définis précédemment et Q représente un atome d'halogène tel que notamment un atome de brome ou un atome de chlore et de préférence un atome de brome. Des bons résultats ont été obtenus par réaction des (thiényl-3′)-2-éthanol ou -3-propanol-1′ avec l'halogénure d'alkyle, d'oxyalkyle, de phényle ou de benzyle approprié. Ainsi, par exemple, le 4-fluorobenzyl-2-(3-thiényl)éthyléther est préparé par réaction de (thiényl-3′)-2-éthanol avec le 1-bromométhyl-4-fluorobenzène dans le tétrahydrofurane en présence d'hydrure de sodium.

La température à laquelle est réalisée cette réaction, est généralement comprise entre 5 et 50°C, de préférence entre 10 et 30°C.

La pression à laquelle est réalisée cette réaction, est généralement comprise entre 1 et 4 bars, de préférence la réaction est réalisée à la pression atmosphérique.

La réaction est réalisée de préférence sous l'atmosphère d'un gaz inerte, tel que notamment l'argon, et en présence d'un solvant, tel que notamment le tétrahydrofurane, et peut être réalisée dans tout réacteur ou appareillage permettant de réunir les conditions susmentionnées.

Un autre objet de l'invention est constitué par les polymères contenant des unités récurrentes dérivées de thiophène substitué selon l'invention.

L'invention concerne à cet effet des polymères contenant des unités récurrentes de formule générale : 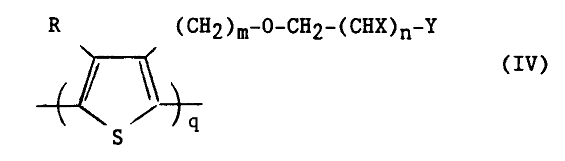
dans laquelle :
- q représente un nombre entier,
- R représente un atome d'hydrogène, un atome d'halogène ou un groupement aliphatique contenant de 1 à 4 atomes de carbone,
- X représente un atome d'hydrogène ou un atome de fluor,
- Y représente un groupement aliphatique ou aromatique au moins partiellement fluoré,
- m représente un nombre entier égal ou supérieur à 2,
- n représente un nombre entier tel que 0 ≦ n ≦ 7.

### Habituellement :

- q représente un nombre entier compris entre 2 et 5000,
- R représente un atome d'hydrogène,
- X représente un atome d'hydrogène ou un atome de fluor,
- Y représente :
   . un radical phényle substitué au moins par un atome de fluor, par un groupement -CF₃, un groupement -CH₂F ou un groupement CHF₂, ou
   . un groupement aliphatique de formule générale -(CF₂)ₚ-CF₃ dans laquelle p représente un nombre entier tel que 0 ≦ p ≦ 16,
- m représente un nombre entier égal ou supérieur à 2,
- n représente un nombre entier tel que 0 ≦ n ≦ 7.

### Généralement :

- q représente un nombre entier compris entre 2 et 3000,
- R représente un atome d'hydrogène,
- X représente un atome d'hydrogène,
- Y représente :
   . un radical phényle substitué par un atome de fluor, par un groupement -CF₃ ou entièrement substitué par des atomes de fluor, ou
   . un groupement aliphatique de formule générale -(CF₂)ₚ-CF₃ dans laquelle p représente un nombre entier tel que 0 ≦ p ≦ 12,
- m représente un nombre entier égal à 2 ou 3,
- n représente un nombre entier tel que 0 ≦ n ≦ 2.

### De manière préférée :

- q représente un nombre entier compris entre 2 et 1000,
- R représente un atome d'hydrogène,
- X représente un atome d'hydrogène,
- Y représente :
   . un radical phényle substitué par un atome de fluor en para, par un groupement -CF₃ en para ou entièrement substitué par des atomes de fluor, ou
   . un groupement aliphatique de formule générale -(CF₂)ₚ-CF₃ dans laquelle p représente un nombre entier tel que 0 ≦ p ≦ 8,
- m est égal à 2,
- n représente un nombre entier égal à 0 ou 1.

### De manière particulièrement préférée :

- q représente un nombre entier compris entre 2 et 500,
- R représente un atome d'hydrogène,
- X représente un atome d'hydrogène,
- Y représente un radical phényle substitué par un atome de fluor en para,
- m est égal à 2,
- n est égal à 0.

Ce produit correspond au polymère du 4-fluorobenzyl-2-(3-thiényl)éthyléther.

Un autre objet de l'invention est constitué par les polymères conducteurs d'électricité contenant un polymère selon l'invention et un agent dopant. L'agent dopant peut être un anion ou un cation, tel que défini ci-après.

La préparation des polymères conducteurs d'électricité selon l'invention peut s'effectuer par voie chimique, en présence d'oxydants par exemple, ou par voie électrochimique. De préférence, on procède par polymérisation électrochimique, généralement dans une cellule d'électrolyse, par oxydation anodique du monomère au sein d'un solvant polaire et en présence d'électrolytes appropriés suivant des techniques conventionnelles telles que décrites notamment dans la demande de brevet français FR-A-2527843.

Selon ces techniques, la concentration en monomères est généralement comprise entre 10⁻³ et 1 mole par litre de solvant.

La température, à laquelle est réalisée la préparation des polymères, est généralement comprise entre 0 et 50°C et, de préférence, entre 5 et 40°C.

La pression, à laquelle est réalisée la préparation des polymères, est généralement voisine de la pression atmosphérique et, de préférence, est égale à la pression atmosphérique.

Comme solvants, on utilise de préférence des solvants polaires possédant des propriétés dissolvantes à la fois vis-à-vis du monomère et de l'électrolyte choisi et stables dans le domaine des potentiels appliqués. Des exemples de solvants utilisables sont l'acétonitrile, le tétrahydrofurane, le chlorure de méthylène, le nitrobenzène et le carbonate de propylène.

Les électrolytes sont généralement choisis parmi les sels conducteurs de formule C⁺A⁻ dans laquelle C⁺ est un cation et dans laquelle A⁻ est un anion.

Le cation C⁺ est choisi de préférence parmi les ions alcalins, les ions R₄N⁺ et R₄P⁺ (R étant un radical alkyle, tel que les radicaux éthyle et butyle par exemple).

L'anion A⁻ est choisi de préférence parmi les ions ClO₄⁻, AsF₆⁻, SbF₆⁻, SO₄²⁻, C₆H₅SO₃⁻, BF₄⁻, PF₆⁻ et CF₃SO₃⁻.

Des électrolytes typiques sont par exemple les fluorophosphates, tels que l'hexafluorophosphate de tétrabutylammonium, les fluoroborates, tels que le tétrafluoroborate de tétraéthylammonium et les perchlorates, tels que le perchlorate de lithium et le perchlorate de tétrabutylammonium.

La concentration en électrolyte est généralement comprise entre 10⁻³ et 1 mole par litre de solvant.

La cellule électrochimique au sein de laquelle peut s'effectuer la polymérisation des monomères selon l'invention peut fonctionner dans des conditions potentiostatiques ou galvanostatiques.

Dans le premier cas (contrôle potentiostatique), la cellule comprend, outre la source de courant externe, trois électrodes dont une électrode de référence de contrôle du potentiel.

Au cours de l'électrolyse, une couche de polymère se dépose sur l'élément conducteur utilisé comme anode de la cellule d'électrolyse. Cette anode peut être réalisée en un métal noble, tel que l'or ou le platine, ou en un autre métal, tel que le cuivre, doré ou platiné, le titane, le nickel ou en un verre conducteur (oxyde d'étain, oxydes d'indium - étain). Après l'électrolyse, on dispose donc en fait d'une électrode constituée par un corps conducteur enrobé par un film de polymère y adhérant et qui contient une certaine proportion de l'anion provenant de l'électrolyte. Le polymère et l'anion forment ainsi un complexe à transfert de charges. La composition chimique du film de polymère peut être représentée par la formule empirique (M⁺Ay⁻)_{q} où M⁺ représente le monomère, A⁻ l'anion ou contre-ion, y la proportion en anion dans le polymère exprimée par unité monomérique (c'est-à-dire le taux de dopage) qui, dans le cas des polymères de l'invention, peut atteindre la valeur de 0,5, et q le degré de polymérisation généralement difficile à déterminer aisément, compte tenu du caractère insoluble du polymère.

La polymérisation électrochimique du monomère s'effectuant sur l'anode de la cellule d'électrolyse, on ne peut obtenir directement une électrode recouverte d'un polymère dopé par des cations.

On peut, pour obtenir une telle cathode, se servir de l'anode obtenue précédemment et lui faire subir une double réduction. Une première réduction électrochimique est possible juste après la polymérisation en laissant l'anode dans la cellule d'électrolyse et en provoquant la décharge de la cellule. Cette décharge provoque l'extraction des anions "dopant" le polymère. On peut ensuite effectuer une seconde réduction sous atmosphère inerte, soit par voie chimique, soit par voie électrochimique. La voie chimique consiste à immerger le polymère dans une solution contenant les cations désirés. Ainsi, pour obtenir un polymère "dopé" par exemple par les cations Li⁺, Na⁺ ou K⁺ (dopage de type "n" auquel les polymères selon l'invention se prêtent particulièrement bien), on peut se servir par exemple d'une solution de naphtalène lithium, de naphtalène sodium ou de naphtalène potassium dans le tétrahydrofurane. La voie électrochimique consiste généralement à placer l'électrode en tant que cathode dans une cellule d'électrolyse contenant en solution les cations désirés. Les cations peuvent être par exemple des ions alcalins tels que ceux mentionnés ci-dessus, de préférence les cations Li⁺ ou K⁺, ou des ions complexes tels que (Bu)₄N⁺ ou (Et)₄N⁺ issus d'un électrolyte (de préférence LiClO₄, KPF₆, (Bu)₄NClO₄ et (Et)₄NClO₄) en solution dans un solvant tel que l'acétonitrile, le tétrahydrofurane ou le propylène carbonate. La concentration en électrolyte dans la solution est généralement comprise entre 10⁻³ et 1 mole pour 1 litre de solvant.

Les polymères conducteurs selon l'invention présentent un ensemble de propriétés tout à fait remarquables qui sont principalement :
- une réversibilité et une stabilité exceptionnelles du cycle d'oxydo-réduction entre leurs formes oxydées et réduites; ainsi, notamment, pour le préféré d'entre ces polymères, la stabilité du cycle d'oxydo-réduction est telle qu'il peut subir jusqu'à 0,5.10⁷ cycles tout en conservant encore 90 % de la charge initiale;
- une variation importante des caractéristiques spectrales obtenue avec une variation de potentiel faible, ce qui rend intéressante et économique leur utilisation en tant que matériau électrochromique;
- une bonne conductivité électrique, généralement comprise entre 1 et 2.10² S.cm⁻¹;
- des absorptions importantes dans le domaine des rayonnements infrarouges proches et à haute fréquence.

Ces propriétés remarquables des polymères conducteurs selon l'invention les rendent particulièrement utilisables pour la réalisation de dispositifs électroconducteurs dont le principe de fonctionnement est basé sur ces propriétés, et qui constituent également un objet de la présente invention.

A titre d'exemples non limitatifs de dispositifs électroconducteurs contenant des polymères conducteurs dérivés des monomères hétérocycliques aromatiques substitués selon l'invention, on peut citer :
- les dispositifs électrochimiques de stockage d'énergie, tels que batteries d'accumulateurs et piles rechargeables ou non, dont les anodes (respectivement les cathodes) sont constituées d'électrodes revêtues de films desdits polymères dopés par des anions (respectivement des cations);
- les dispositifs électrochromiques basés sur la modification du spectre optique desdits polymères selon leur état d'oxydation, qui se manifeste lors des cycles d'oxydation et de réduction des films de polymères déposés sur les anodes (respectivement les cathodes) de ces dispositifs lors de la charge et de la décharge; à titre d'exemples de pareils dispositifs électrochromiques, on peut citer les écrans d'affichage (display), les dispositifs optoélectroniques, les mémoires et commutateurs optiques, les dispositifs d'absorption des ondes électromagnétiques.

L'invention est illustrée par les exemples suivants.

### Exemple 1

Dans un tricol de 1 l placé sous argon, on introduit successivement 120 ml de tétrahydrofurane desséché sur sodium, 120 mmol d'hydrure de sodium en suspension à 60 % dans de l'huile minérale, puis en 10 minutes une solution de 100 mmol de (thiényl-3′)-2-éthanol dans 60 ml de tétrahydrofurane.

La suspension est maintenue 30 min sous agitation à 20°C, sous atmosphère d'argon.

On ajoute ensuite en 30 min une solution de 130 mmol de 1-bromométhyl-4-fluorobenzène dans 120 ml de tétrahydrofurane.

La suspension est portée au reflux sous atmosphère d'argon pendant 4 heures.

La suspension refroidie à température ambiante est traitée par 120 ml d'éther et 100 ml d'eau.

La phase organique est décantée, puis lavée trois fois avec 100 ml d'eau, ensuite séchée sur sulfate de magnésium et évaporée à sec sous vide.

Le produit brut est purifié par chromatographie (SiO₂/heptane/AcOEt) puis est distillé sous vide.

Le rendement en 4-fluorobenzyl-2-(3′-thiényl)éthyléther s'élève à 75 % calculé en mole pour cent.

### Exemple 2

La synthèse du polymère est effectuée dans une cellule thermostatée à un compartiment contenant :
- 2.10⁻¹ mole de monomère préparé comme à l'exemple 1,
- 3.10⁻² mole d'hexafluorophosphate de tétrabutylammonium (fourni par Fluka),
- 25 ml de nitrobenzène distillé.

Les dépôts de polymère sont réalisés à 5°C, sous atmosphère d'argon, après dégazage de la solution par barbotage d'argon. Pour les caractéristiques électrochimiques, le polymère est déposé sur une électrode de platine massif d'une surface de 0,7 cm² polie. La quantité de charges utilisées est de 100 mC/cm² et la densité de courant est égale à 2 mA/cm², ce qui conduit à un dépôt de polymère d'environ 0,45 µ d'épaisseur. La cathode est constituée d'un fil de platine et l'électrode de référence est une électrode au calomel saturée.

La conductivité électrique du polymère ainsi obtenu est de l'ordre de 5 S.cm⁻¹.

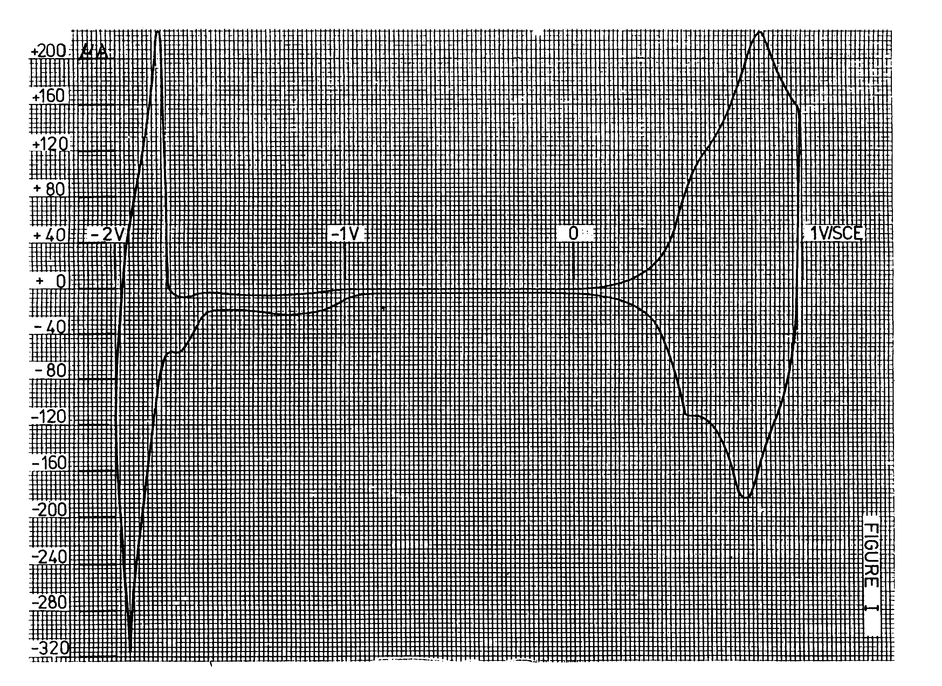

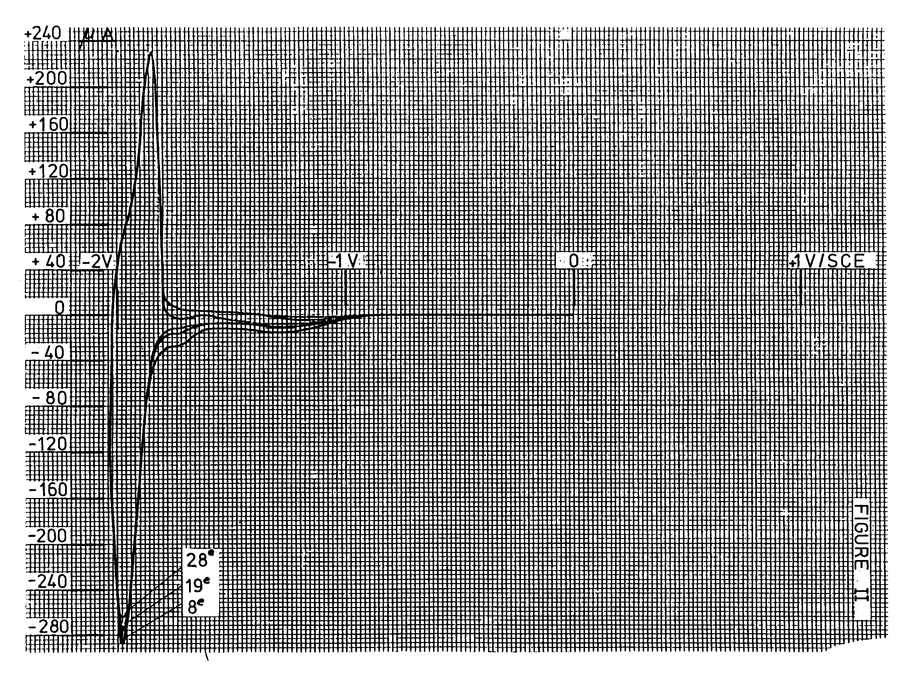

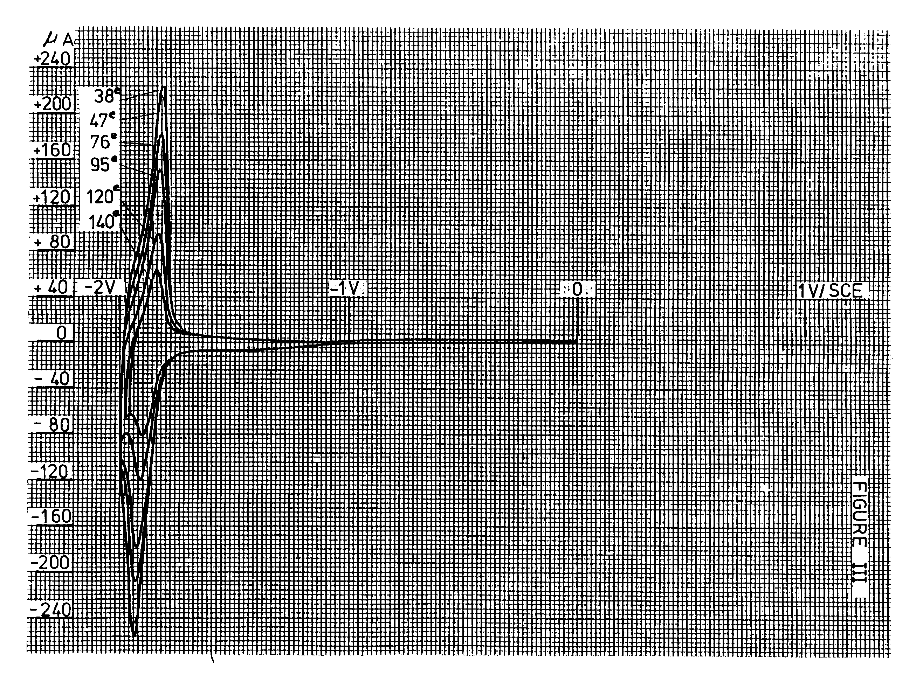

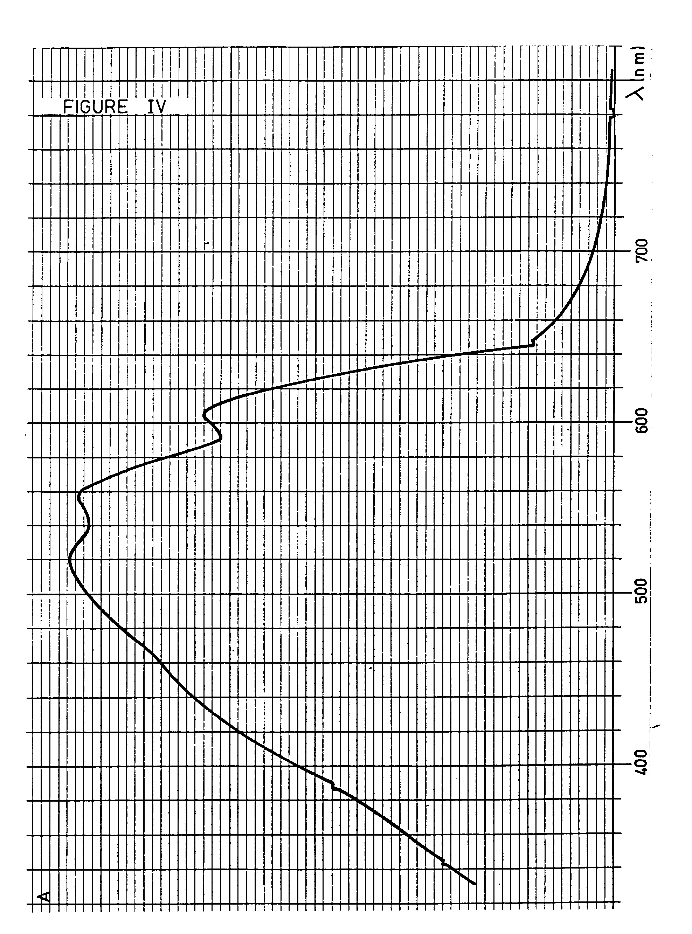

Le spectre d'absorption du polymère dédopé est représenté à la figure IV. L'absorption est importante entre 450 et 600 nm et très faible au delà de 600 nm. La longueur d'onde (λ) en abscisse est mesurée en nanomètre (nm), l'absorbance (A) est donnée en ordonnée (unité arbitraire).

Les propriétés électrochimiques des polymères ont été mesurées à partir du voltammogramme cyclique enregistré au moyen d'un potentiostat PAR modèle 173 et à partir des pics d'intensité enregistrés. La figure I représente un voltammogramme réalisé avec une vitesse de balayage de 100 mV/s dans 0,1 mole de perchlorate de tétraéthylammonium mis en solution dans CH₃CN, l'unité de l'abscisse est le volt (V), l'unité de l'ordonnée est le microampère (µA).

Les rapports Ipₐ/Ip_{c} entre les intensités de courant d'oxydation (Ipₐ) et de reduction (Ip_{c}) (pour le système p) sont d'environ 1.

Le dopage "p" du polymère est très réversible. La charge échangée après 10⁶ cycles est encore de 95 %.

On travaille à l'aide d'impulsions de potentiel situées entre 0 et 0,7 volt par rapport au fil d'argent et de 50 ms de durée, dans des conditions qui permettent de voir le contraste optique (épaisseur du film : 0,10 µ).

Le cyclage rédox est effectué dans un milieu électrolytique constitué de carbonate de propylène contenant 5.10⁻¹ mole de perchlorate de lithium anhydre.

Les tests de dopage "n" ont été réalisés dans le carbonate de propylène contenant 0,1 mole de perchlorate de tétraéthylammonium.

En ne procédant pas selon des conditions particulières (dégazage à l'argon et solvants séchés sur P₂O₅) pour empêcher l'oxydation des polymères dopés n formés, on obtient la succession de cyclages indiqués aux figures II et III. Ceux-ci ont été réalisés avec une vitesse de balayage de 100 mV/s dans 0,1 mole de perchlorate de tétraéthylammonium mis en solution dans CH₃CN, les chiffres (8e, 19e, 28e pour la figure II et 38e, ..., 140e pour la figure III) représentent le nombre de cycles effectués. L'unité de l'abscisse est le volt, l'unité de l'ordonnée est le microampère.

## Revendications (Revendications pour l'(les) Etat(s) contractant(s) suivant(s): AT, BE, CH, DE, FR, GB, GR, IT, LI, LU, NL, SE)

1. Thiophènes substitués de formule générale : 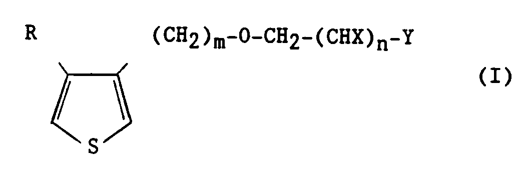 caractérisés en ce que :
- R représente un atome d'hydrogène, un atome d'halogène ou un groupement aliphatique contenant de 1 à 4 atomes de carbone,
- X représente un atome d'hydrogène ou un atome de fluor,
- Y représente un groupement aliphatique ou aromatique au moins partiellement fluoré,
- m représente un nombre entier égal ou supérieur à 2,
- n représente un nombre entier tel que 0 ≦ n ≦ 7.

2. Thiophène selon la revendication 1, caractérisé en ce qu'il s'agit du 4-fluorobenzyl-2-(3-thiényl)éthyléther.

3. Procédé pour la fabrication de thiophènes substitués selon la revendication 1 ou 2, caractérisé en ce que l'on fait réagir un composé de formule générale : 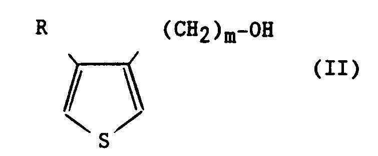 avec un composé de formule générale :
Q-CH₂-(CHX)ₙ-Y (III)
dans laquelle Q représente un atome d'halogène.

4. Polymères contenant des unités récurrentes de formule générale : 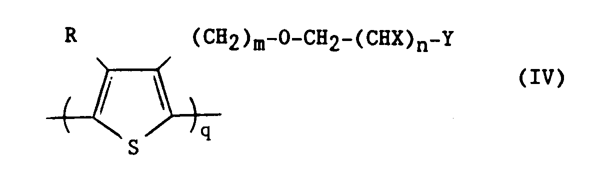 dans laquelle :
- q représente un nombre entier,
- R représente un atome d'hydrogène, un atome d'halogène ou un groupement aliphatique contenant de 1 à 4 atomes de carbone,
- X représente un atome d'hydrogène ou un atome de fluor,
- Y représente un groupement aliphatique ou aromatique au moins partiellement fluoré,
- m représente un nombre entier égal ou supérieur à 2,
- n représente un nombre entier tel que 0 ≦ n ≦ 7.

5. Polymère selon la revendication 4, caractérisé en ce qu'il s'agit du polymère du 4-fluorobenzyl-2-(3-thiényl)éthyléther.

6. Polymère conducteur d'électricité contenant un polymère selon la revendication 4 ou 5 et un agent dopant.

7. Procédé pour la fabrication de polymères conducteurs d'électricité selon la revendication 6 caractérisé en ce que l'on polymérise électrochimiquement, par oxydation anodique au sein d'un solvant polaire et en présence d'un électrolyte approprié, un monomère choisi parmi les thiophènes substitués selon la revendication 1 ou 2.

8. Dispositifs électroconducteurs contenant un polymère conducteur d'électricité selon la revendication 6.

9. Dispositifs électrochimiques de stockage d'énergie dont les anodes (respectivement les cathodes) sont constituées d'électrodes revêtues de films de polymères selon la revendication 4 ou 5 dopés par des anions (respectivement des cations).

10. Dispositifs électrochromiques dont les anodes (respectivement les cathodes) sont revêtues de films de polymères selon la revendication 6.

11. Dispositifs d'absorption des rayonnements infrarouges proches et à haute fréquence à base de polymères selon la revendication 6.

## Revendications (Revendications pour l'(les) Etat(s) contractant(s) suivant(s): ES)

1. Procédé pour la fabrication de thiophènes substitués de formule générale : 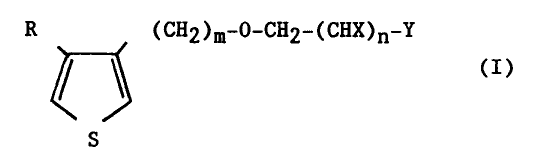 dans laquelle :
- R représente un atome d'hydrogène, un atome d'halogène ou un groupement aliphatique contenant de 1 à 4 atomes de carbone,
- X représente un atome d'hydrogène ou un atome de fluor,
- Y représente un groupement aliphatique ou aromatique au moins partiellement fluoré,
- m représente un nombre entier égal ou supérieur à 2,
- n représente un nombre entier tel que 0 ≦ n ≦ 7, caractérisé en ce que l'on fait réagir un composé de formule générale : 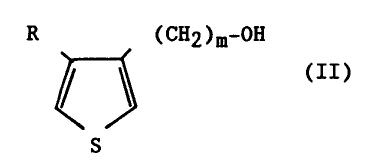
avec un composé de formule générale :
Q-CH₂-(CHX)ₙ-Y (III)
dans laquelle Q représente un atome d'halogène.

2. Procédé selon la revendication 1, caractérisé en ce que l'on fait réagir le (thiényl-3')-2-éthanol avec le 1-bromométhyl-4-fluorobenzène dans le tétrahydrofurane en présence d'hydrure de sodium.

3. Procédé pour la fabrication de polymères conducteurs d'électricité contenant un agent dopant et un polymère qui contient des unités récurrentes de formule générale : 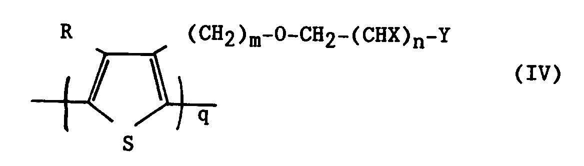 dans laquelle :
- q représente un nombre entier,
- R représente un atome d'hydrogène, un atome d'halogène ou un groupement aliphatique contenant de 1 à 4 atomes de carbone,
- X représente un atome d'hydrogène ou un atome de fluor,
- Y représente un groupement aliphatique ou aromatique au moins partiellement fluoré,
- m représente un nombre entier égal ou supérieur à 2,
- n représente un nombre entier tel que 0 ≦ n ≦ 7, caractérisé en ce que l'on polymérise électrochimiquement, par oxydation anodique au sein d'un solvant polaire et en présence d'un électrolyte approprié, un monomère choisi parmi les thiophènes substitués obtenus selon la revendication 1 ou 2.

4. Procédé selon la revendication 3, caractérisé en ce que l'on met en oeuvre un solvant polaire choisi parmi l'acétonitrile, le tétrahydrofurane, le chlorure de méthylène, le nitrobenzène et le carbonate de propylène.

5. Procédé selon la revendication 3 ou 4, caractérisé en ce que l'on met en oeuvre un électrolyte choisi parmi les fluorophosphates, tels que l'hexafluorophosphate de tétrabutylammonium, les fluoroborates, tels que le tétrafluoroborate de tétraéthylammonium et les perchlorates, tels que le perchlorate de lithium et le perchlorate de tétrabutylammonium.

6. Dispositifs électroconducteurs contenant un polymère conducteur d'électricité obtenu selon la revendication 3, 4 ou 5.

7. Dispositifs électrochimiques de stockage d'énergie dont les anodes (respectivement les cathodes) sont constituées d'électrodes revêtues de films de polymères obtenus selon la revendication 3, 4 ou 5 dopés par des anions (respectivement des cations).

8. Dispositifs électrochromiques dont les anodes (respectivement les cathodes) sont revêtues de films de polymères obtenus selon la revendication 3, 4 ou 5.

9. Dispositifs d'absorption des rayonnements infrarouges proches et à haute fréquence à base de polymères obtenus selon la revendication 3, 4 ou 5.

## Claims (Claims for the following Contracting State(s): AT, BE, CH, DE, FR, GB, GR, IT, LI, LU, NL, SE)

1. Substituted thiophenes of general formula: 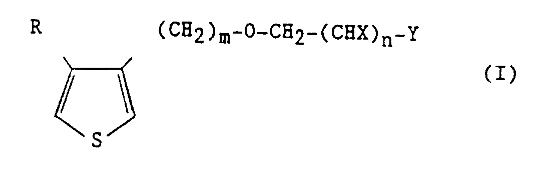 characterized in that:
- R represents a hydrogen atom, a halogen atom or an aliphatic group containing from 1 to 4 carbon atoms,
- X represents a hydrogen atom or a fluorine atom,
- Y represents an at least partially fluorinated aliphatic or aromatic group,
- m represents an integer equal to or greater than 2, and
- n represents an integer such that 0 ≦ n ≦ 7.

2. Thiophene according to Claim 1, characterized in that it is 4-fluorobenzyl 2-(3-thienyl)ethyl ether.

3. Process for the production of substituted thiophenes according to Claim 1 or 2, characterized in that a compound of general formula: 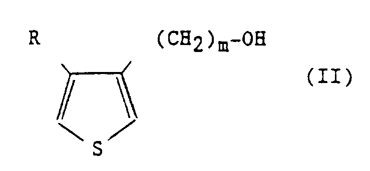 is reacted with a compound of general formula:
Q-CH₂-(CHX)ₙ-Y (III)
in which Q represents a halogen atom.

4. Polymers containing recurring units of general formula: 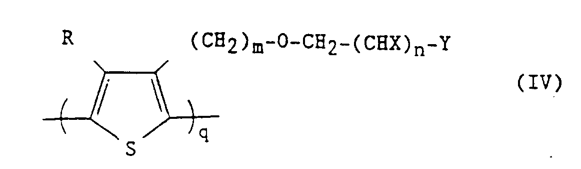 in which:
- q represents an integer,
- R represents a hydrogen atom, a halogen atom or an aliphatic group containing from 1 to 4 carbon atoms,
- X represents a hydrogen atom or a fluorine atom,
- Y represents an at least partially fluorinated aliphatic or aromatic group,
- m represents an integer equal to or greater than 2, and
- n represents an integer such that 0 ≦ n ≦ 7.

5. Polymer according to Claim 4, characterized in that it is the polymer of 4-fluorobenzyl 2-(3-thienyl)-ethyl ether.

6. Electrically conductive polymer containing a polymer according to Claim 4 or 5 and a doping agent.

7. Process for the production of electrically conductive polymers according to Claim 6, characterized in that a monomer chosen from the substituted thiophenes according to Claim 1 or 2 is electrochemically polymerized by anodic oxidation in a polar solvent and in the presence of an appropriate electrolyte.

8. Electrically conductive devices containing an electrically conductive polymer according to Claim 6.

9. Electrochemical devices for storing energy in which the anodes (or the cathodes) are constituted by electrodes coated with films of polymers according to Claim 4 or 5 doped with anions (or cations).

10. Electrochromic devices in which the anodes (or the cathodes) are covered with films of polymers according to Claim 6.

11. Near-infrared and high-frequency radiation absorption devices based on polymers according to Claim 6.

## Claims (Claims for the following Contracting State(s): ES)

1. Process for the manufacture of substituted thiophenes of general formula: 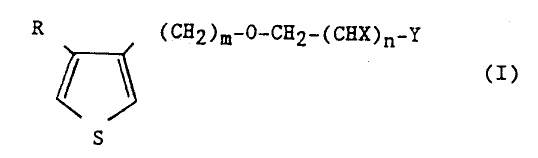 in which:
- R represents a hydrogen atom, a halogen atom or an aliphatic group containing from 1 to 4 carbon atoms,
- X represents a hydrogen atom or a fluorine atom,
- Y represents an at least partially fluorinated aliphatic or aromatic group,
- m represents an integer equal to or greater than 2, and
- n represents an integer such that 0 ≦ n ≦ 7, characterized in that a compound of general formula: 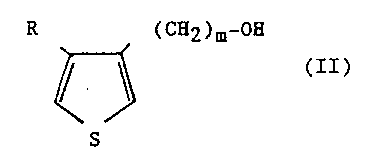
is reacted with a compound of general formula:
Q-CH₂-(CHX)ₙ-Y (III)
in which Q represents a halogen atom.

2. Process according to Claim 1, characterized in that 2-(3'-thienyl)ethanol is reacted with 1-bromomethyl-4-fluorobenzene in tetrahydrofuran in the presence of sodium hydride.

3. Process for the manufacture of electrically conductive polymers containing a doping agent and a polymer which contains recurring units of general formula: 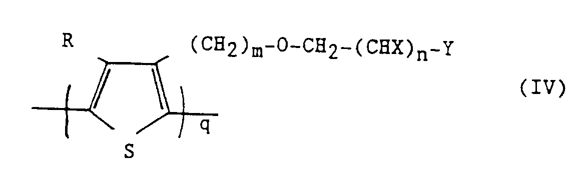 in which:
- q represents an integer,
- R represents a hydrogen atom, a halogen atom or an aliphatic group containing from 1 to 4 carbon atoms,
- X represents a hydrogen atom or a fluorine atom,
- Y represents an at least partially fluorinated aliphatic or aromatic group,
- m represents an integer equal to or greater than 2, and
- n represents an integer such that 0 ≦ n ≦ 7, characterized in that a monomer chosen from the substituted thiophenes obtained according to Claim 1 or 2 is electrochemically polymerized by anodic oxidation in a polar solvent and in the presence of an appropriate electrolyte.

4. Process according to Claim 3, characterized in that a polar solvent chosen from acetonitrile, tetrahydrofuran, methylene chloride, nitrobenzene and propylene carbonate is used.

5. Process according to Claim 3 or 4, characterized in that an electrolyte chosen from fluorophosphates such as tetrabutylammonium hexafluorophosphate, fluoroborates such as tetraethylammonium tetrafluoroborate and perchlorates such as lithium perchlorate and tetrabutylammonium perchlorate is used.

6. Electrically conductive devices containing an electrically conductive polymer obtained according to Claim 3, 4 or 5.

7. Electrochemical devices for storing energy in which the anodes (or the cathodes) are constituted by electrodes coated with films of polymers obtained according to Claim 3, 4 or 5 doped with anions (or cations).

8. Electrochromic devices in which the anodes (or the cathodes) are covered with films of polymers obtained according to Claim 3, 4 or 5.

9. Near-infrared and high-frequency radiation absorption devices based on polymers obtained according to Claim 3, 4 or 5.

## Patentansprüche (Patentansprüche für folgende(n) Vertragsstaat(en): AT, BE, CH, DE, FR, GB, GR, IT, LI, LU, NL, SE)

1. Substituierte Thiophene der allgemeinen Formel: 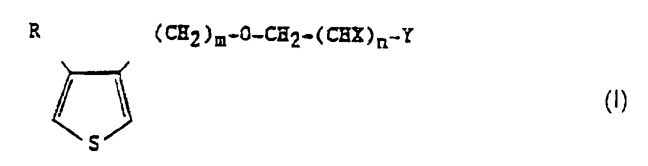 dadurch gekennzeichnet, daß:
- R ein Wasserstoffatom, ein Halogenatom oder eine aliphatische Gruppe mit 1 bis 4 Kohlenstoffatomen darstellt,
- X ein Wasserstoffatom oder ein Fluoratom darstellt,
- Y eine wenigstens teilweise fluorierte aliphatische oder aromatische Gruppe darstellt,
- m eine ganze Zahl gleich oder größer 2 darstellt,
- n eine ganze Zahl wie 0 ≦ n ≦ 7 darstellt.

2. Thiophen gemäß Anspruch 1, dadurch gekennzeichnet, daß es sich um 4-Fluorbenzyl-2-(3-thienyl)ethylether handelt.

3. Verfahren zur Herstellung substituierter Thiophene gemäß Anspruch 1 oder 2, dadurch gekennzeichnet, daß man eine Verbindung der allgemeinen Formel: 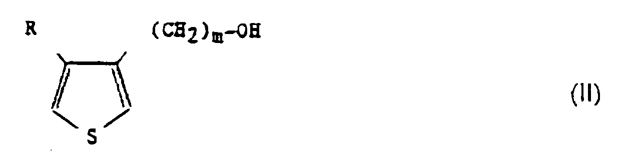 mit einer Verbindung der allgemeinen Formel:
Q-CH₂-(CHX)ₙ-Y (III)
worin Q ein Halogenatom darstellt, umsetzt.

4. Polymere, die wiederkehrende Einheiten der allgemeinen Formel: 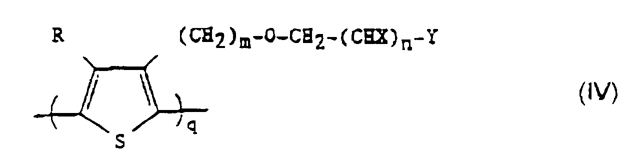 enthalten, worin:
- q eine ganze Zahl darstellt,
- R ein Wasserstoffatom, ein Halogenatom oder eine aliphatische Gruppe mit 1 bis 4 Kohlenstoffatomen darstellt,
- X ein Wasserstoffatom oder ein Fluoratom darstellt,
- Y eine wenigstens teilweise fluorierte aliphatische oder aromatische Gruppe darstellt,
- m eine ganze Zahl gleich oder größer 2 darstellt,
- n eine ganze Zahl wie 0 ≦ n ≦ 7 darstellt.

5. Polymer gemäß Anspruch 4, dadurch gekennzeichnet, daß es sich um das Polymer des 4-Fluorbenzyl-2-(3-thienyl)ethylethers handelt.

6. Elektrisch leitfähiges Polymer, das ein Polymer gemäß Anspruch 4 oder 5 und ein Dotierungsmittel enthält.

7. Verfahren zur Herstellung elektrisch leitfähiger Polymere gemäß Anspruch 6, dadurch gekennzeichnet, daß man ein aus den substituierten Thiophenen gemäß Anspruch 1 oder 2 ausgewähltes Monomer durch anodische Oxidation in einem polaren Lösungsmittel und in Gegenwart eines geeigneten Elektrolyten elektrochemisch polymerisiert.

8. Elektrisch leitende Vorrichtungen, die ein elektrisch leitfähiges Polymer gemäß Anspruch 6 enthalten.

9. Elektrochemische Vorrichtungen zur Energiespeicherung, deren Anoden (beziehungsweise deren Kathoden) aus Elektroden gebildet werden, die mit Filmen der Polymere gemäß Anspruch 4 oder 5, die mit Anionen (beziehungsweise Kationen) dotiert sind, beschichtet sind.

10. Elektrochrome Vorrichtungen, deren Anoden (beziehungsweise Kathoden) mit Filmen der Polymere gemäß Anspruch 6 beschichtet sind.

11. Vorrichtungen zur Absorption von Strahlung des nahen Infrarot und von hoher Frequenz auf der Basis von Polymeren gemäß Anspruch 6.

## Patentansprüche (Patentansprüche für folgende(n) Vertragsstaat(en): ES)

1. Verfahren zur Herstellung substituierter Thiophene der allgemeinen Formel: 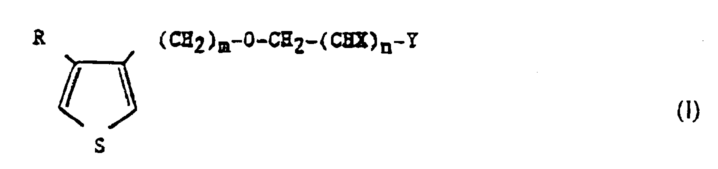 worin:
- R ein Wasserstoffatom, ein Halogenatom oder eine aliphatische Gruppe mit 1 bis 4 Kohlenstoffatomen darstellt,
- X ein Wasserstoffatom oder ein Fluoratom darstellt,
- Y eine wenigstens teilweise fluorierte aliphatische oder aromatische Gruppe darstellt,
- m eine ganze Zahl gleich oder größer 2 darstellt,
- n eine ganze Zahl wie 0 ≦ n ≦ 7 darstellt, dadurch gekennzeichnet, daß man eine Verbindung der allgemeinen Formel: 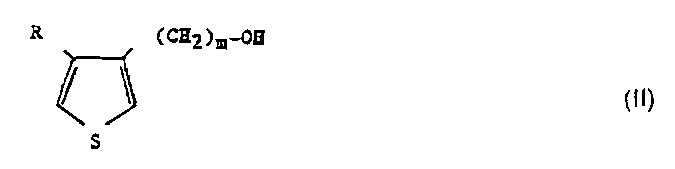
mit einer Verbindung der allgemeinen Formel:
Q-CH₂-(CHX)ₙ-Y (III)
worin Q ein Halogenatom darstellt, umsetzt.

2. Verfahren gemäß Anspruch 1, dadurch gekennzeichnet, daß man (Thienyl-3')-2-ethanol mit 1-Brommethyl-4-fluorbenzol in Tetrahydrofuran in Gegenwart von Natriumhydrid umsetzt.

3. Verfahren zur Herstellung elektrisch leitfähiger Polymere, die ein Dotierungsmittel und ein Polymer enthalten, welches wiederkehrende Einheiten der allgemeinen Formel: 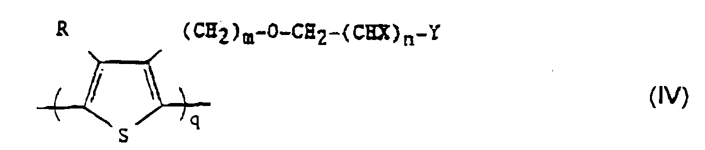 enthält, worin:
- q eine ganze Zahl darstellt,
- R ein Wasserstoffatom, ein Halogenatom oder eine aliphatische Gruppe mit 1 bis 4 Kohlenstoffatomen darstellt,
- X ein Wasserstoffatom oder ein Fluoratom darstellt,
- Y eine wenigstens teilweise fluorierte aliphatische oder aromatische Gruppe darstellt,
- m eine ganze Zahl gleich oder größer 2 darstellt,
- n eine ganze Zahl wie 0 ≦ n ≦ 7 darstellt,
dadurch gekennzeichnet, daß man ein aus den gemäß Anspruch 1 oder 2 erhaltenen substituierten Thiophenen ausgewähltes Monomer durch anodische Oxidation in einem polaren Lösungsmittel und in Gegenwart eines geeigneten Elektrolyten elektrochemisch polymerisiert.

4. Verfahren gemäß Anspruch 3, dadurch gekennzeichnet, daß man ein aus Acetonitril, Tetrahydrofuran, Methylenchlorid, Nitrobenzol und Propylencarbonat ausgewähltes polares Lösungsmittel einsetzt.

5. Verfahren gemäß Anspruch 3 oder 4, dadurch gekennzeichnet, daß man einen Elektrolyten einsetzt, der ausgewählt ist aus den Fluorophosphaten, wie das Tetrabutylammoniumhexafluorophosphat, den Fluoroboraten, wie das Tetraethylammoniumtetrafluoroborat, und den Perchloraten, wie das Lithiumperchlorat und das Tetrabutylammoniumperchlorat.

6. Elektrisch leitende Vorrichtungen, die ein gemäß Anspruch 3, 4 oder 5 erhaltenes elektrisch leitfähiges Polymer enthalten.

7. Elektrochemische Vorrichtungen zur Energiespeicherung, deren Anoden (beziehungsweise deren Kathoden) aus Elektroden gebildet werden, die mit Filmen der gemäß Anspruch 3, 4 oder 5 erhaltenen Polymere, die mit Anionen (beziehungsweise Kationen) dotiert sind, beschichtet sind.

8. Elektrochrome Vorrichtungen, deren Anoden (beziehungsweise Kathoden) mit Filmen der gemäß Anspruch 3, 4 oder 5 erhaltenen Polymere beschichtet sind.

9. Vorrichtungen zur Absorption von Strahlung des nahen Infrarot und von hoher Frequenz auf der Basis von gemäß Anspruch 3, 4 oder 5 erhaltenen Polymeren.
